(19) Europäisches Patentamt
European Patent Office
Office européen des brevets

(11) Veröffentlichungsnummer: **0 286 043 B1**

# (12) EUROPÄISCHE PATENTSCHRIFT

(45) Veröffentlichungstag der Patentschrift: **23.06.93**

(51) Int. Cl.$^5$: **G02F 1/137**, C09K 19/58, C09K 19/12, C09K 19/34, C09K 19/20

(21) Anmeldenummer: **88105354.0**

(22) Anmeldetag: **02.04.88**

## (54) Verwendung von Verbindungen mit chiralem, polarem Molekülteil als Komponenten in Flüssigkristall-Mischungen für die Ausnutzung des elektroklinen Effektes.

(30) Priorität: **07.04.87 DE 3711362**

(43) Veröffentlichungstag der Anmeldung:
**12.10.88 Patentblatt 88/41**

(45) Bekanntmachung des Hinweises auf die Patenterteilung:
**23.06.93 Patentblatt 93/25**

(84) Benannte Vertragsstaaten:
**AT BE CH DE ES FR GB IT LI NL SE**

(56) Entgegenhaltungen:
**EP-A- 0 263 225**
**EP-A- 0 263 437**
**EP-A- 0 275 522**

**LIQUID CRYSTALS, Band 2, Nr. 6, 1987, Seiten 825-831; CH. BAHR et al.: "Optical and dielectric investigations on the electroclinic effect exhibited by a ferroelectric liquid crystal with high spontaneous polarization"**

(73) Patentinhaber: **HOECHST AKTIENGESELLSCHAFT**
**Postfach 80 03 20**
**W-6230 Frankfurt am Main 80(DE)**

(72) Erfinder: **Bahr, Christian**
**Hohenzollerndamm 189**
**W-1000 Berlin 31(DE)**
Erfinder: **Heppke, Gerd, Prof. Dr.**
**Johann-Georg-Strasse 3**
**W-1000 Berlin 31(DE)**
Erfinder: **Lötzsch, Detlef**
**Lessingstrasse 10**
**W-1000 Berlin 21(DE)**
Erfinder: **Dübal, Hans-Rolf, Dr.**
**Heuhohlweg 6**
**W-6240 Königstein/Taunus(DE)**
Erfinder: **Günther, Dieter, Dr.**
**Nachtigallenweg 1A**
**W-6233 Kelkheim (Taunus)(DE)**
Erfinder: **Hemmerling, Wolfgang, Dr.**
**Billtalstrasse 32**
**W-6231 Sulzbach (Taunus)(DE)**

**EP 0 286 043 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

PHYSICAL REVIEW A, Band 19, Nr. 1, Januar 1979, Seiten 338-347, The American Physical Society; S. GAROFF et al.: "Electroclinic effect at the A-C phase change in a chiral smectic liquid crystal"

MOLECULAR CRYSTALS AND LIOUID CRYSTALS, Letters Section, Band 4, Nr. 2, 1986, Seiten 31-37, Gordon and Breach Science Publishers, New York, US; CH. BAHR et al.: "Ferroelectric liquid crystals with high spontaneous polarisation"

Erfinder: **Müller, Ingrid, Dr.**
**Am Pfingstbrunnen 1**
**W-6238 Hofheim am Taunus(DE)**
Erfinder: **Wingen, Rainer, Dr.**
**Rotkäppchenweg 10**
**W-6234 Hattersheim am Main(DE)**

**Beschreibung**

Die ungewöhnliche Kombination von anisotropen und fluiden Eigenschaften der Flüssigkristalle haben zu ihrer Verwendung in einer Vielzahl von elektro-optischen Schalt-und Anzeigevorrichtungen geführt. Dabei können ihre elektrischen, magnetischen, elastischen oder thermischen Eigenschaften zu Orientierungsänderungen benutzt werden. Optische Effekte lassen sich dann mit Hilfe ihrer Doppelbrechung ("birefringence mode"), der Einlagerung dichroitisch absorbierender Farbstoffmoleküle ("guest-host mode") oder der Lichtstreuung erzielen.

Seit einigen Jahren ist der sogenannte elektrokline Effekt in der chiralen smektischen A-Phase ($S_A^*$ -Phase) bekannt, der sich zur Lichtmodulation ausnutzen läßt (S. Garoff und R.B. Meyer, Phys. Rev. Lett. 38, 848 (1977)). Wenn man ein elektrisches Feld parallel zu den Schichten einer chiralen $S_A$-Phase ($S_A^*$ -Phase) anlegt, so neigen sich die Moleküle in der an sich orthogonalen Phase. Der Neigungswinkel $\theta$ zwischen dem Direktor $\hat{n}$ und der Schichtennormalen $\hat{z}$ ist dem angelegten Feld E proportional. Der elektrokline Koeffizient ($d\theta/dE$) gibt die Stärke der linearen Kopplung zwischen der Neigungskoordinate und dem Feld an.

In der von Clark und Lagerwall vorgeschlagenen "bookshelf"-Anordnung (N.A. Clark und S.T. Lagerwall, Appl. Phys. Lett. 36, 899 (1980)) kann der elektro-optische Effekt ferroelektrischer Flüssigkristalle ausgenutzt werden. Deren Charakteristika wie Bistabilität, spontane Polarisation und Phasenverdrillung sind den hier besprochenen orthogonalen Phasen wesensfremd. Der elektrokline Effekt geht von einem im feldfreien Raum monostabilen Zustand aus und ergibt beim Anlegen eines Feldes eine für kleine Winkel lineare und für große Winkel schwach gekrümmte elektro-optische Kennlinie. Die Kennlinie des ferroelektrischen Schaltens ist dagegen stark nichtlinear.

Der elektrokline Effekt kann als eine kontinuierlich durch das Feld gesteuerte Auslenkung des Neigungswinkels aufgefaßt werden.

Für die praktische Ausnutzung des elektroklinen Effekts in elektro-optischen Schalt- und Anzeigeelementen sind flüssigkristalline Medien, die eine $S_A^*$ , $S_B^*$ oder $S_E^*$ Phase bilden umso besser geeignet, je größer der elektrokline Koeffizient $d\theta/dE$ und je höher die elektrokline Grenzfrequenz $f_G$ ist.

Es besteht daher großes Interesse an Substanzen oder Substanzgemischen, die $S_A^*$ , $S_B^*$ oder $S_E^*$ Phasen mit einem großen elektroklinen Koeffizienten und einer hohen elektroklinen Grenzfrequenz bilden.

Es wurde nun gefunden, daß man auf der Ausnutzung des elektroklinen Effekts in flüssigkristallinen Medien in der $S_A^*$ , $S_B^*$ oder $S_E^*$ Phase beruhende elektro-optische Schalt- und Anzeigelemente mit besonders guten Eigenschaften erhält, wenn man als flüssigkristallines Medium alleine oder in Mischung mit anderen Komponenten Verbindungen verwendet, die eine polare chirale Gruppierung (Molekülteil) aus der Gruppe

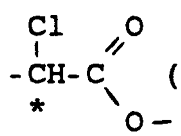 (I) , 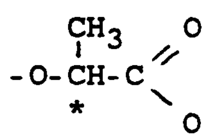 (II) , 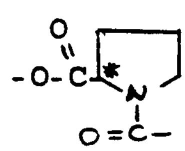 (III),

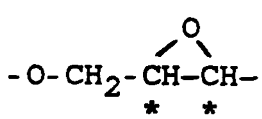 (IV) oder 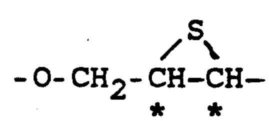 (V)

enthalten.

Gegenstand der Erfindung sind auch flüssigkristalline Medien in der $S_A^*$ , $S_B^*$ oder $S_E^*$ Phase, die einen Gehalt an den genannten Verbindungen aufweisen, sowie elektro-optische Schalt- und Anzeigeelementen, die solche Medien enthalten.

Die gemäß der Erfindung zu verwendenden Verbindungen sind Derivate von $\alpha$-Chlorcarbonsäuren (I), $\alpha$-Hydroxypropionsäure (II), Prolin (III), von Epoxyverbindungen (IV) oder deren Thioanalogen (V). Es handelt sich um Verbindungen, die beschrieben sind in

(I) Ch. Bahr und G. Heppke, Mol. Cryst. Liq. Cryst. Lett. 4, 31 (1986) und in dort zitierter Literatur

(II) DE-A 36 38 119

(III) DE-A 36 44 522
(IV,V) DE-A 36 33 968
Sie entsprechen der allgemeinen Formel

$R^1$ - $Z^*$ - $R^2$ (A),

in der-$Z^*$-einen der chiralen Reste I-V bedeutet, und in der $R^1$ und/oder $R^2$ übliche mesogene Reste sind. Mesogene Reste sind Reste, die der Verbindung solche Eigenschaften verleihen, daß sie als Verbindung oder im Gemisch mit bestimmten Cokomponenten eine Flüssigkristallphase zeigt, d.h. eine enantiotrope (Klärtemperatur > Schmelztemperatur) oder monotrope (Klärtemperatur < Schmelztemperatur) Mesophasenbildung erwarten läßt. Zu den mesogenen Resten zählen dabei insbesondere gleiche oder unterschiedliche Reste der allgemeinen Formel (B):

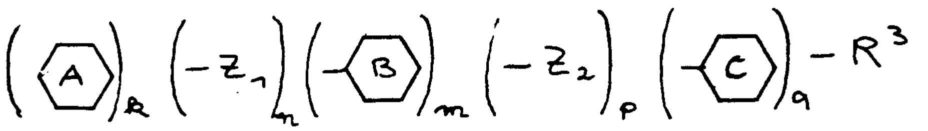 (B)

worin die Substituenten, Bausteine und Indices folgende Bedeutung haben

| | |
|---|---|
| k, n, m, p, q | Null oder 1, jedoch p = Null wenn m = Null und k + m + q mindestens 1 |
| $Z_1$ | CO-O, O-CO, $(CH_2)_2$, $OCH_2$, $CH_2O$, CO-S, S-CO |
| $Z_2$ | $Z_1$ oder $CH_2$, N = N, N = N(O), CH = N, N = CH |

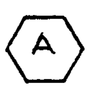 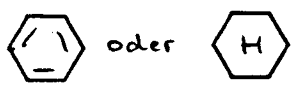

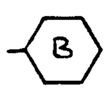 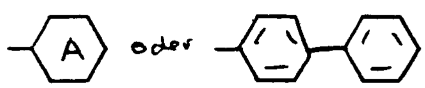

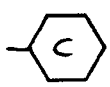 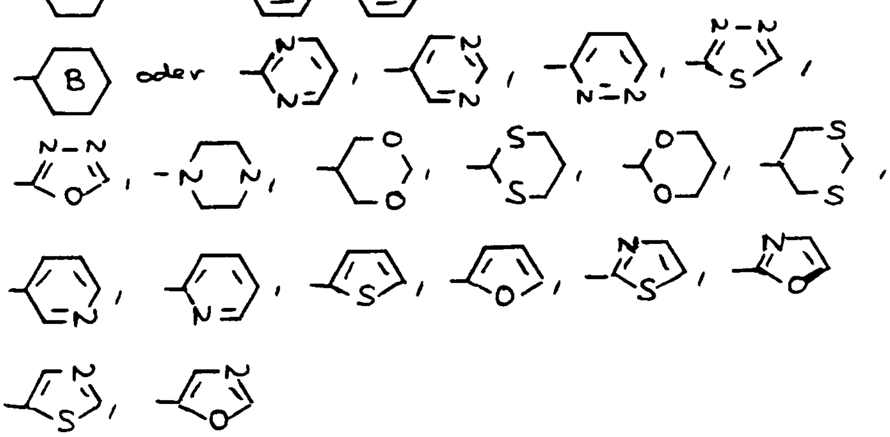

| | |
|---|---|
| $R_3$ | $C_rH_{2r+1}$, $O-C_rH_{2r+1}$, $S-C_rH_{2r+1}$, $CO-C_rH_{2r+1}$, $O-CO-C_rH_{2r+1}$ oder $CO-O-C_rH_{2r+1}$ wobei r eine ganze Zahl von 1 bis 20 ist. |

Sofern einer der Reste $R^1$, $R^2$ nicht-mesogen ist, dann bedeutet dieser bevorzugt eine geradkettige oder verzweigte durch F, Cl, Br oder CN substituierte oder unsubstituierte Alkylgruppe mit 1 bis 20 C-Atomen, in der eine oder zwei nicht-benachbarte und nicht direkt der Gruppierung (I) bis (V) benachbarte $-CH_2$-Gruppen ersetzt sein können durch -O-, -S-, -CO-, -O-CO-, -CO-O-, -O-CS-, -CO-S- und/oder -CH=CH-, oder die eine Endgruppe $-CH=CH_2$ oder $-CH=C(CH_3)_2$ oder

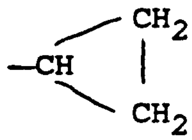

aufweist.

Diese Verbindungen bilden zum Teil selbst orthogonale ($S_A^*$ , $S_B^*$ oder $S_E^*$ ) flüssigkristalline Phasen aus und zeigen dann bereits für sich einen ausgeprägten elektroklinen Effekt. In diesem Fall können sie für sich alleine in der orthogonalen flüssigkristallinen Phase als Medium in entsprechenden Schalt- oder Anzeigeelementen verwendet werden.

Sie können jedoch auch anderen bekannten Komponenten, die orthogonale flüssigkristalline Phasen bilden, als chirale Dotierstoffe zugesetzt werden, insbesondere enthalten die Mischungen dann 0,1 bis 70 Gew.-% an dem oder den Dotierstoff(en). Sofern diese (Stamm-)Komponente selbst nicht chiral ist, induziert der chirale Dotierstoff den elektroklinen Effekt. Ist diese (Stamm-)Komponente selbst chiral, so erhöht der chirale Dotierstoff den elektroklinen Koeffizienten, also die Amplitude der elektroklinen Bewegung.

Die Vielzahl der Variationsmöglichkeiten bezüglich der chiralen Gruppierung -Z*- und der Substituenten $R^1$ und $R^2$ in (A) gestattet es dabei, die Dotierstoffe hinsichtlich des Phasenbereiches und der Löslichkeitsverhältnisse genau auf eine vorgegebenen orthogonale (Stamm-)Komponente abzustimmen.

Die Erfindung wird anhand der Beispiele näher erläutert. Die Meßergebnisse in Tabelle 3 wurden an einer Zelle mit einer Schichtstärke des flüssigkristallinen Mediums von 2 $\mu$m gewonnen. Die Schicht war durch Scherung oder durch Oberflächenbehandlung planar ausgerichtet. Die Ergebnisse zeigen, daß bereits mit mäßigen Feldstärken große Neigungswinkel $\theta$ erhalten werden, was zu großen Kontrasten führt. Außerdem wurden hohe Grenzfrequenzen des elektroklinen Effekts und damit kurze Schaltzeiten erzielt. Ferner zeigt sich, daß durch die mit der Einführung der chiralen, polaren Gruppe -Z*- verbundenen Symmetriebrechung in einer orthogonal-smektischen Phase in jedem Fall der elektroklinen Effekt induziert wird. Wählt man in Formel (A) ein smektogenes Gerüst, so erreicht man eine effiziente Koppelung des chiralen Zentrums an die orthogonal-smektische Phase. Durch die spezielle Wahl der chiralen Gruppierungen I-V für -Z*- in Formel (A) erhält man eine starke Koppelung zum anliegenden elektrische Feld.

Tabelle 1: Strukturvariationen der Formel (A)

| Nr. | $R^1$ | $-Z^*-$ | $R^2$ |
|---|---|---|---|
| 1 | 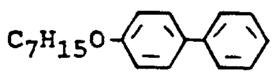 | -OOC-C*HCl- | $CH(CH_3)_2$ |
| 2 | 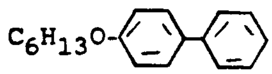 | -OOC-C*HCl- | 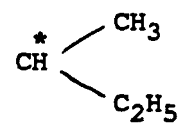 |
| 3 | 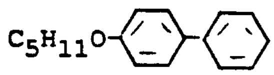 | -OOC-C*HCl- | $CH_2-CH(CH_3)_2$ |
| 4 | 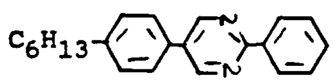 | -OOC-C*HCl- | 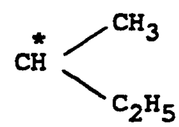 |
| 5 | 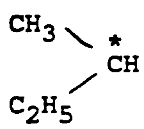 | 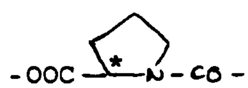 | 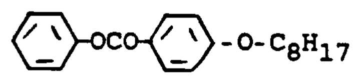 |
| 6 | 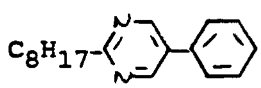 | 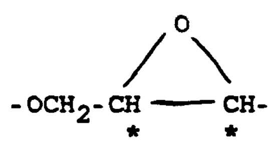 | $C_3H_7$ |
| 7 | $C_4H_9$ | 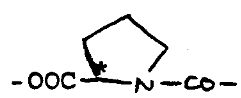 | 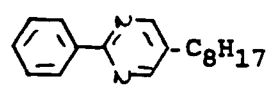 |

| Nr. | $R^1$ | $-Z^*-$ | $R^2$ |
|---|---|---|---|
| 8 | 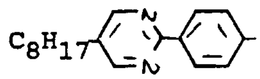 | -OOC-$\overset{*}{C}$HCl- | 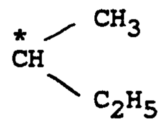 |
| 9 | 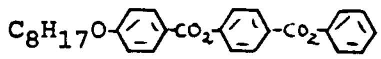 | 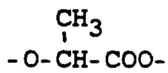 | $C_2H_5$ |
| 10 | 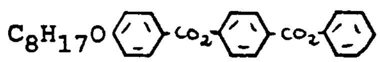 | 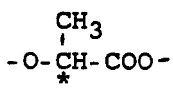 | $C_8H_{17}$ |
| 11 | 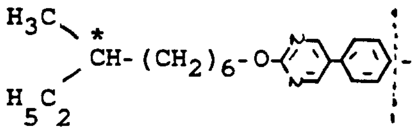 | -OOC-$\overset{*}{C}$HCl- | 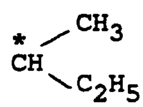 |
| 12 | 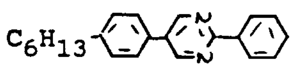 | -OOC-$\overset{*}{C}$HCl- | $C_4H_9$ |
| 13 | 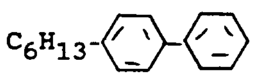 | -OOC-$\overset{*}{C}$HCl- | $C_2H_5$ |

Tabelle 2: Phasenfolgen der Beispiele

| Nr. | $K^*$ | $S_G^*$ | $S_E^*$ | $S_B^*$ | $S_C^*$ | $S_A^*$ | $N^*$ | $I^*$ | Bemerkg. |
|---|---|---|---|---|---|---|---|---|---|
| 1 | · 71 | · 72 | - | - | · 73,4 | · 81,5 | - | · | a |
| 2 | · 45 | · 50 | - | - | · 54,5 | · 64 | - | · | a |
| 3 | · 58 | · | · 63,5 | · 72,5 | - | · 75 | - | · | a |
| 4 | · 123 | - | - | - | · 132,3 | · 161 | - | · | a |
| 5 | · 30 | - | - | - | · 48 | · 53-60 | - | · | b |
| 6 | · 55 | - | - | · 90 | - | · 102 | - | · | a |
| 7 | · | | | | · | · 30-45 | | · | c |
| 8 | · 13 | - | - | - | · 49 | · 60 | - | · | d |
| 9 | · 35 | - | - | - | · 65 | · 75 | - | · | c |
| 10 | · 71 | - | - | - | - | · 91 | · 99 | · | a |
| 11 | · 6 | - | - | - | · 52 | · 55 | - | · | e |
| 12 | ·112 | - | - | - | · 135 | ·176 | - | · | a |
| 13 | · 60 | ($S_4$) | 75($S_3$) 80 | · 89 | - | ·93 | - | · | g |
| 14 | · 10 | - | - | - | · 38 | · | - | · | f |

Bemerkungen zur Tabelle 2:

a als Reinsubstanz gemessen
b in Mischung mit der racematischen Verbindung

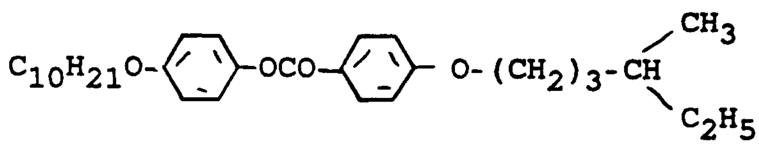

gemessen, der Molenbruch der chiralen Substanz betrug x = 0,2
c wie b, nur betrug der Molenbruch der chiralen Substanz in diesem Fall x = 0,1

d in Mischung mit dem Racemat

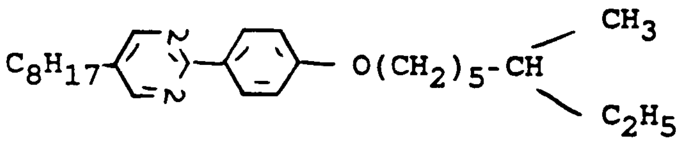

gemessen, der Molenbruch der chiralen Substanz betrug x = 0,1
e wie d, nur betrug der Molenbruch der chiralen Substanz in diesem Fall x = 0,175
f Mischung aus dem Substanzbeispiel Nr. 2 (x = 0,5) und unter Bemerkung d) angegebenen Racemat (x = 0,5).

g Mischung aus Substanzbeispiel Nr. 13 (x = 0,6) und Substanzbeispiel Nr. 3 (x = 0,4).

Tabelle 3: Phys. Meßdaten zum elektroklinen Effekt

| Beispiel | t [°C] | Phase | (dθ/dE) [$10^{-9}$ rad m/V] | $f_G$ [MHz] |
|---|---|---|---|---|
| 1 | 74,5 | $S_A^*$ | 30 | 0,9 |
| | 76,2 | $S_A^*$ | 10 | 1,8 |
| | 79,3 | $S_A^*$ | 5 | 3,3 |
| 2 | 55,5 | $S_A^*$ | 15 | 0,4 |
| | 57,5 | $S_A^*$ | 9 | 0,75 |
| | 60,5 | $S_A^*$ | 4 | 1,3 |
| 3 | 62,5 | $S_E^*$ | 5 | 0,0025 |
| | 64,3 | $S_B^*$ | 7 | 0,036 |
| | 70,5 | $S_B^*$ | 5,3 | 0,23 |
| | 72,1 | $S_B^*$ | 5,2 | 0,63 |
| | 72,5 | $S_A^*$ | 3,8 | > 3 |
| 4 | 133,2 | $S_A^*$ | 50 | 0,87 |
| | 135,3 | $S_A^*$ | 25 | 1,6 |
| | 142,3 | $S_A^*$ | 6 | 3,4 |

| Beispiel | t | Phase | (dθ/dE) | $f_G$ |
|---|---|---|---|---|
| 5 | 48,1 | $S_A^*$ | 7,2 | 0,002 |
| | 49,1 | $S_A^*$ | 2,3 | 0,02 |
| | 50,1 | $S_A^*$ | 1,1 | 0,035 |
| 6 | 60 | $S_B^*$ | 2,0 | - |
| | 70 | $S_B^*$ | 1,6 | - |
| | 80 | $S_B^*$ | 1,1 | - |
| | 95 | $S_A^*$ | kein Effekt meßbar | |
| | 100 | $S_A^*$ | | |
| 7 | 30-40 | $S_A^*$ /$I^*$ | elektrokliner Effekt optisch nachgewiesen | |
| 8 | 48 | $S_A^*$ | 1,5 | > 0,5 |
| 9 | 72 | $S_A^*$ | elektrokliner Effekt optisch nachgewiesen | |
| | 70,5 | $S_A^*$ | 0,8 | - |

| Beispiel | t | Phase | (dθ/dE) | $f_G$ |
|---|---|---|---|---|
| 10 | 85 | $S_A^*$ | 0,8 | - |
| | 80 | $S_A^*$ | 1,2 | - |
| | 75 | $S_A^*$ | 1,6 | - |
| 11 | 52,8 | $S_A^*$ | 7 | - |
| | 52,0 | $S_A^*$ | 11 | - |
| 12 | 138,4 | $S_A^*$ | 25 | 3,2 |
| | 143,3 | $S_A^*$ | 10 | 6,3 |
| | 153,3 | $S_A^*$ | 4 | ~10 |
| 13 | 83,9 | $S_B^*$ | 10 | 0,01 |
| | 86,0 | $S_B^*$ | 8 | 0,056 |
| | 88,2 | $S_B^*$ | 7 | 0,230 |
| | 91 | $S_A^*$ | 3 | > 3 |
| 14 | 38,2 | $S_A^*$ | 45 | 0,11 |
| | 42,2 | $S_A^*$ | 14 | 0,29 |
| | 47,2 | $S_A^*$ | 5 | 0,79 |

## Patentansprüche

1. Verwendung von Verbindungen, die eine polare chirale Gruppierung -Z*- aus der Gruppe

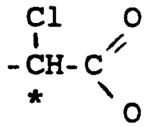 (I) , 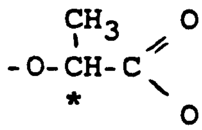 (II) , 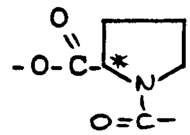 (III),

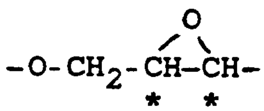 (IV) oder 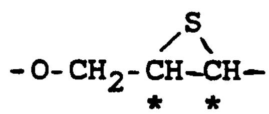 (V)

enthalten, alleine oder in Mischung mit anderen Komponenten in auf der Ausnutzung des elektroklinen Effekts in flüssigkristallinen Medien beruhenden elektrooptischen Schalt- und Anzeigeelementen, bei denen im Betriebszustand das flüssigkristalline Medium in der $S_A^*$ , $S_B^*$ oder $S_E^*$ Phase vorliegt.

2. Verwendung nach Anspruch 1, dadurch gekennzeichnet, daß die Verbindungen der allgemeinen Formel (A)

$R^1$ - Z* - $R^2$ (A)

entsprechen, wobei $R^1$ und/oder $R^2$ mesogene organische Reste sind oder wenn einer der beiden Reste nichtmesogen ist, dieser eine geradkettige oder verzweigte durch F, Cl, Br oder CN substituierte oder unsubstituierte Alkylgruppe mit 1 bis 20 C-Atomen bedeutet, in der eine oder zwei nicht-benachbarte und nicht direkt der Gruppierung (I) bis (V) benachbarte $-CH_2$-Gruppen ersetzt sein können durch -O-, -S-, -CO-, -O-CO-, -CO-O-, -O-CS-, -CO-S- und/oder -CH=CH-, oder die eine Endgruppe $-CH=CH_2$ oder $-CH=C(CH_3)_2$ oder

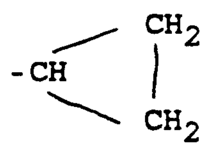

aufweist.

3. Flüssigkristallines Medium in der $S_A^*$ , $S_B^*$ oder $S_E^*$ Phase, gekennzeichnet durch einen Gehalt an mindestens einer Verbindung gemäß Anspruch 1, die eine der polaren chiralen Gruppen (I) bis (V) aufweist.

4. Elektrooptisches Schalt- oder Anzeigeelement, dessen Funktion auf der Ausnutzung des elektroklinen Effektes beruht, und das ein flüssigkristallines Medium enthält, dadurch gekennzeichnet, daß das Schalt- oder Anzeigeelement ein flüssigkristallines Medium nach Anspruch 3 enthält.

## Claims

**1.** The use of a compound which contains a polar chiral grouping -Z*- from the group comprising

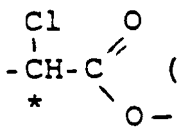 (I) , 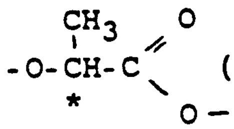 (II) , 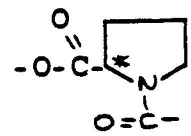 (III),

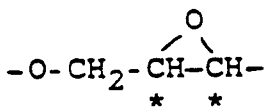 (IV) or 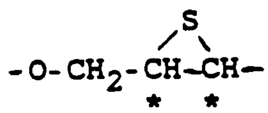 (V)

alone or mixed with other components in electrooptical switching and indicating elements which are based on the exploitation of the electroclinic effect in liquid-crystalline media and in which the liquid-crystalline medium is present in the $S_A^*$ , $S_B^*$ or $S_E^*$ phase in the operational state.

**2.** The use as claimed in claim 1, where in the compound corresponds to the formula (A)

$R^1$ - Z* - $R^2$ (A),

$R^1$ and/or $R^2$ being mesogenic organic radicals or, if one of the two radicals is not mesogenic, it denotes a straight-chain or branched alkyl group containing 1 to 20 carbon atoms which is substituted or unsubstituted by F, Cl, Br or CN and in which one or two non-adjacent $-CH_2-$ groups, which are also not directly adjacent to the groupings (I) to (V), may be replaced by -O-, -S-, -CO-, -O-CO-, -CO-O-, -O-CS-, -CO-S- and/or -CH=CH-, or which has a terminal end group $-CH=CH_2$ or $-CH=C(CH_3)_2$ or

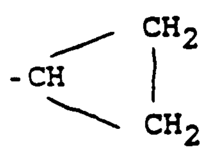

**3.** A liquid-crystalline medium in the $S_A^*$ , $S_B^*$ or $S_E^*$ phase which has a content of at least one compound as claimed in claim 1 which has one of the polar chiral groups (I) to (V).

**4.** An electrooptical switching or indicating element whose operation is based on the exploitation of the electroclinic effect and which contains a liquid-crystalline medium, wherein the switching or indicating element contains a liquid-crystalline medium as claimed in claim 3.

## Revendications

1. Utilisation de composés contenant un groupement chiral polaire -Z*- choisi parmi l'ensemble

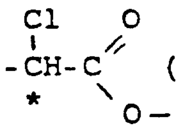 (I) , 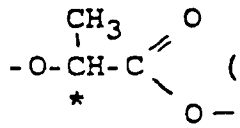 (II) , 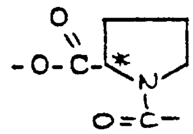 (III),

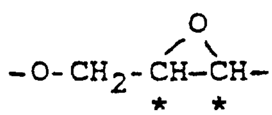 (IV) ou 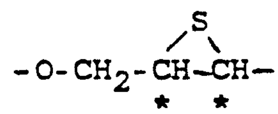 (V)

seuls ou en mélange avec d'autres composants, dans des éléments de commutation ou d'affichage optoélectroniques se fondant sur l'utilisation de l'effet électrocline dans des milieux mésomorphes, où, en mode d'exploitation, le milieu mésomorphe se trouve en phase $S_A^*$, $S_B^*$ ou $S_E^*$.

2. Utilisation selon la revendication 1, caractérisée en ce que les composés correspondent à la formule (A)

$R^1$ - Z* - $R^2$ (A)

dans laquelle $R^1$ et/ou $R^2$ sont des radicaux organiques mésogènes ou encore, quand l'un des deux radicaux n'est pas mésogène, il représente un groupe alkyle à chaîne droite ou ramifiée, éventuellement substitué par F, Cl, Br ou CN et ayant de 1 à 20 atomes de carbone, groupe dans lequel un ou deux groupes $-CH_2-$ non voisins, et non directement voisins du groupement (I) à (V), peuvent être remplacés par -O-, -S-, -CO-, -O-CO, -CO-O-, -O-CS-, -CO-S- et/ou -CH = CH-, ou encore qui comporte le groupe terminal $-CH=CH_2$ ou $-CH=C(CH_3)_2$ ou

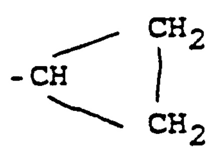

3. Milieu mésomorphe en phase $S_A^*$, $S_B^*$ ou $S_E^*$, caractérisé en ce qu'il contient au moins un composé de formule 1 contenant l'un des groupes chiraux polaires (I) à (V).

4. Elément de commutation ou d'affichage optoélectronique dont la fonction se fonde sur l'utilisation de l'effet électrocline, et qui contient un milieu mésomorphe, caractérisé en ce que l'élément de commutation ou d'affichage contient un milieu mésomorphe selon la revendication 3.